Europäisches Patentamt

European Patent Office

Office européen des brevets

(19)

(11) Veröffentlichungsnummer: **0 163 869**
**B1**

(12)

# EUROPÄISCHE PATENTSCHRIFT

(45) Veröffentlichungstag der Patentschrift:
**03.06.87**

(21) Anmeldenummer: **85104498.2**

(22) Anmeldetag: **13.04.85**

(51) Int. Cl.⁴: **C 22 C  19/07**, C 22 C  30/00,
A 61 K  6/04

(54) Verwendung einer Kobalt-Chrom-Legierung für Dentalprothesen.

(30) Priorität: **05.05.84  DE 3416608**

(43) Veröffentlichungstag der Anmeldung:
**11.12.85 Patentblatt 85/50**

(45) Bekanntmachung des Hinweises auf die Patenterteilung:
**03.06.87 Patentblatt 87/23**

(84) Benannte Vertragsstaaten:
**AT BE CH DE FR GB IT LI LU NL SE**

(56) Entgegenhaltungen:
**DE - C - 642 040**
**FR - A - 2 516 832**
**US - A - 4 263 045**

(73) Patentinhaber: **Degussa Aktiengesellschaft,
Weissfrauenstrasse 9, D-6000 Frankfurt am Main 1 (DE)**

(72) Erfinder: **Nawaz, M. H. A., Dr., Hinter den Gärten 6,
D-7531 Neulingen (DE)**
Erfinder: **Stümke, Manfred, Dr. Dipl.-Phys., Am
Walsweg 21, D-7530 Pforzheim (DE)**

### Beschreibung

Die Erfindung betrifft eine Kobalt-Chrom-Legierung zur Herstellung von Dentalprothesen, die vorzugsweise mit Dentalkeramik beschichtet werden können.

In der Zahnheilkunde werden Legierungen zur Herstellung von Kronen- und Brückengerüsten verwendet, die anschliessend mit dentalkeramischen Massen verblendet werden. Diese Keramikmassen erfüllen in einer hervorragenden Weise die ästhetischen Forderungen, die an einen Zahnersatz gestellt werden. Dabei ist es notwendig, dass der Wärmeausdehnungskoeffizient der hierfür verwendeten Legierungen dem der Dentalkeramikmassen angepasst ist, da sonst während oder nach der Herstellung der Prothese Risse in der Keramikverblendung entstehen. Hierzu geeignete Legierungen müssen ausserdem eine ausreichend niedrige Liquidustemperatur und gute Gusseigenschaften aufweisen. Es ist bekannt, Gold- und Palladiumlegierungen zur Herstellung solcher Brücken- und Kronengerüste zu verwenden. Die bekannten Edelmetallegierungen erfüllen die geforderten Bedingungen sehr gut, sind aber wegen ihres hohen Edelmetallgehaltes sehr teuer.

Es hat daher nicht an Versuchen gefehlt, billigere Unedelmetallegierungen auf Kobalt-Nickel-Chrom- und Kobalt-Chrombasis zu entwickeln, die in ihren Eigenschaften den bekannten Edelmetall-Legierungen gleichkommen.

So werden in der US-PS-40 10 048 Nickel-Chrom-Legierungen beschrieben, die ausserdem noch Molybdän, Eisen, Aluminium und Silizium enthalten. Nickelhaltige Legierungen haben jedoch den Nachteil, dass Nickel allergische Reaktionen beim Patienten und beim Labor- und Praxispersonal hervorrufen kann.

Es ist daher versucht worden, nickelfreie Legierungen für prothetische Teile zu entwickeln, die mit keramischen Massen oder Kunststoffen verblendet werden können. So sind in letzter Zeit Kobalt-Chrom-Molybdän-Gusslegierungen bekanntgeworden, die jedoch nicht zunderbeständig sind und daher nicht mit Keramik verblendet werden können. Ausserdem sind sie sauerstoffempfindlich und müssen deswegen im Vakuum geschmolzen werden.

Es war daher Aufgabe der vorliegenden Erfindung, Legierungen auf der Basis Kobalt-Chrom für Dentalprothesen zu entwickeln, die möglichst kein Nickel enthalten, mit keramischen Massen und Kunststoffen verblendbar sind, sich in normaler Atmosphäre in üblichen Giessgeräten schmelzen lassen und keine grösseren Mengen an Schlacken im Schmelztiegel bilden.

Diese Aufgabe wurde erfindungsgemäss durch die Verwendung einer Kobalt-Chrom-Legierung gelöst, die neben Kobalt als Rest aus 23 bis 36% Chrom, 16 bis 22% Eisen, 1 bis 10% Molybdän, 0,05 bis 3% Mangan, 0,05 bis 1% Titan und 0 bis 1% Silizium besteht.

Vorzugsweise verwendet man Legierungen, die neben Kobalt als Rest 28 bis 33% Chrom, 17 bis 19% Eisen, 2 bis 6% Molybdän, 0,5 bis 1,5% Mangan, 0,1 bis 0,5% Titan und 0 bis 0,5% Silizium enthalten.

Nickel kann in diesen Legierungen in Gehalten bis etwa 0,5% enthalten sein und aus Verunreinigungen der Legierungskomponenten, insbesondere des Kobalts herrühren.

Die Summe der Elemente Mangan, Titan und Silizium sollte dabei nicht über 2% liegen.

Solche Legierungen lassen sich gut mit keramischen Massen und Kunststoffen verblenden, sind in normaler Atmosphäre in den üblichen Giessgeräten schmelzbar und bilden nur unwesentliche mengen Schlacken.

Legierungen ähnlicher Zusammensetzung sind für andere Anwendungsbereiche bekannt, wo gute Korrosions- und Abriebbeständigkeit verlangt werden. Solche Legierungen werden z.B. verwendet zur Beschichtung von Teilen, deren Oberfläche korrosiven Medien und/oder hohen Abriebkräften ausgesetzt ist. Sie werden ausserdem zur Herstellung von Strangpressformen verwendet, wo gute Thermoschockbeständigkeit und Hochtemperaturfestigkeit verlangt werden.

Wie nunmehr gefunden wurde, besitzen diese Legierungen in dem genannten Zusammensetzungsbereich Wärmeausdehnungskoeffizienten, die denen der üblichen Dentalkeramiken angepasst sind. Durch ihr niedriges und enges Schmelzintervall lassen sie sich z.B. mit der Propan-Sauerstoff-Flamme gut schmelzen und giessen.

Darüber hinaus können diese Legierungen auch als Gusslegierungen zur Herstellung von Platten und Gerüsten für herausnehmbaren Zahnersatz verwendet werden, da ihre technischen Daten den Anforderungen für solche Legierungen entsprechen.

Ihre guten mechanischen Eigenschaften und das günstige Verarbeitungsverhalten erlauben weiterhin ihre Verwendung als Gusslegierung für die Herstellung von Kronen- und Brückengerüsten zur Kunststoffverblendung.

Folgende Beispiele sollen die Vorteile dieser Legierungen bei der Verwendung für Dentalprothesen näher erläutern. Dabei wurden die Legierungen mit einer Propan-Sauerstoff-Flamme sowie in Dentalgiessgeräten mit Hochfrequenz-Induktion geschmolzen und vergossen. Anschliessend wurden Brückengerüste mit bis zu vierzehn Gliedern hergestellt und diese mit marktüblichen dentalkeramischen Massen verblendet. Weder Risse noch Sprünge wurden in der Keramik festgestellt. Die Giessfähigkeit der Legierungen und die Ergebnisse der Verblendversuche waren durchaus befriedigend.

Beispiel 1:

Zusammensetzung: 31,0% Chrom, 18,8% Eisen, 3,0% Molybdän, 0,9% Mangan, 0,2% Titan, Rest Kobalt einschliesslich herstellungsbedingter Verunreinigungen.

| | |
|---|---|
| Schmelzintervall (°C): | 1395-1345 |
| 0,2%-Dehngrenze $R_{p0,2}$ (N/mm²): | 520- 550 |
| Zugfestigkeit $R_m$ (N/mm²): | 630- 660 |

Dehnung A$_5$ (%): 3- 5
Vickershärte HV 10: 320- 350

Beispiel 2:

Zusammensetzung: 29,4% Chrom, 18,4% Eisen, 5,8% Molybdän, 0,8% Mangan, 0,1% Titan, Rest Kobalt einschliesslich herstellungsbedingter Verunreinigungen.

Schmelzintervall (°C): 1385-1340
0,2%-Dehngrenze R$_{p0,2}$ (N/mm²): 720- 740
Zugfestigkeit R$_m$ (N/mm²): 760- 780
Dehnung A$_5$ (%): 0,3- 1
Vickershärte HV 10: 400- 420

Beispiel 3:

Zusammensetzung: 29,2% Chrom, 18,3% Eisen, 5,7% Molybdän, 0,8% Mangan, 0,1% Titan, 0,5% Si, Rest Kobalt einschliesslich herstellungsbedingter Verunreinigungen.

Schmelzintervall (°C): 1380-1340
0,2%-Dehngrenze R$_{p0,2}$ (N/mm²): 680- 700
Zugfestigkeit R$_m$ (N/mm²): 700- 720
Dehnung A$_5$ (%): 0,3- 1
Vickershärte HV 10: 400- 420

Beispiel 4:

Zusammensetzung: 25,5% Chrom, 19,0% Eisen, 8,0% Molybdän, 0,8% Mangan, 0,1% Titan, Rest Kobalt einschliesslich herstellungsbedingter Verunreinigungen.

Schmelzintervall (°C): 1375-1300
0,2%-Dehngrenze R$_{p0,2}$ (N/mm²): 600- 630
Zugfestigkeit R$_m$ (N/mm²): 720- 790
Dehnung A$_5$ (%): 5- 8
Vickershärte HV 10: 300

## Patentansprüche

1. Verwendung einer Kobalt-Chrom-Legierung aus 23 bis 36% Chrom, 16 bis 22% Eisen, 1 bis 10% Molybdän, 0,05 bis 3% Mangan, 0,05 bis 1% Titan, 0 bis 1% Silizium, Rest Kobalt, zur Herstellung von Dentalprothesen.

2. Verwendung einer Legierung nach Anspruch 1, *dadurch gekennzeichnet,* dass sie aus 29 bis 33% Chrom, 17 bis 19% Eisen, 2 bis 6% Molybdän, 0,5 bis 1,5% Mangan, 0,1 bis 0,5% Titan, 0 bis 0,5% Silizium, Rest Kobalt, besteht.

3. Verwendung einer Legierung nach Anspruch 1 und 2, *dadurch gekennzeichnet,* dass die Summe der Elemente Mangan, Silizium und Titan nicht über 2% liegt.

## Claims

1. Use of a cobalt chromium alloy composed of from 23 to 36% of chromium, from 16 to 22% of iron, from 1 to 10% of molybdenum, from 0.05 to 3% of manganese, from 0.05 to 1% of titanium, from 0 to 1% of silicon, the remainder being cobalt, for the production of dental prostheses.

2. Use of an alloy according to claim 1, characterised in that it is composed of from 29 to 33% of chromium, from 17 to 19% of iron, from 2 to 6% of molybdenum, from 0.5 to 1.5% of manganese, from 0.1 to 0.5% of titanium, from 0 to 0.5% of silicon, the remainder being cobalt.

3. Use of an alloy according to claims 1 and 2, characterised in that the sum of the elements manganese, silicon and titanium does not exceed 2%.

## Revendications

1. Utilisation d'un alliage cobalt-chrome fait de 23 à 36% en poids de chrome, 16 à 22% de fer, 1 à 10% de molybdène, 0,05 à 3% de manganèse, 0,05 à 1% de titane, 0 à 1% de silicium, le reste étant du cobalt, pour la fabrication de prothèses dentaires.

2. Utilisation d'un alliage suivant la revendication 1, caractérisée en ce qu'il est constitué de 29 à 33% de chrome, 17 à 19% de fer, 2 à 6% de molybdène, 0,5 à 1,5% de manganèse, 0,1 à 0,5% de titane, 0 à 0,5% de silicium.

3. Utilisation d'un alliage suivant les revendications 1 et 2, caractérisée en ce que la somme des éléments manganèse, silicium et titane ne dépasse pas 2%.